# EUROPÄISCHE PATENTSCHRIFT

(11) **EP 0 673 945 B1**
(45) Veröffentlichungstag und Bekanntmachung des Hinweises auf die Patenterteilung: **06.09.2000**
(21) Anmeldenummer: 95103555.9
(22) Anmeldetag: 13.03.1995
(51) Int. Cl.: C07F 9/54, C07C 255/10, C07C 69/65

(54) **Herstellung eines Wittigestersalzes**
Preparation of an ester salt of wittig
Préparation d'un ester sel de wittig

(30) Priorität: 23.03.1994 CH 86894
(43) Veröffentlichungstag der Anmeldung: 27.09.1995
(73) Patentinhaber: F. HOFFMANN-LA ROCHE AG, 4002 Basel (CH)
(72) Erfinder: Burdet, Bruno, F-68390 Baldersheim (FR); Rüttimann, August, CH-4144 Arlesheim (CH); Santer, Jean-Marie, F-68300 St. Louis (FR); Siegfried, Theodor, CH-4125 Riehen (CH)
(74) Vertreter: Kellenberger, Marcus, Dr.

(56) Entgegenhaltungen:
- EP-A- 0 110 329
- EP-A- 0 294 774
- JUSTUS LIEBIGS ANN. CHEM. (JLACBF,00754617);77; (7); PP.1146-59, BASF A.-G.;HAUPTLAB.; LUDWIGSHAFEN/RHEIN; GER. Nuerrenbach A*** et al 'Oxidation von Phosphor-Yliden mit Hydroperoxiden: Ein neuer und ergiebiger Weg zu symmetrischen Carotinoiden.'
- CHEMICAL ABSTRACTS, vol. 119, no. 21, 22. November 1993, Columbus, Ohio, US; abstract no. 225949, YOSHIHARA H ET AL 'Preparation of aminopyrazole derivatives' & JP-A-93 140 113 (SUMITOMO CHEMICAL CO;JAPAN ) 8. Juni 1993

## Beschreibung

Die vorliegende Erfindung betrifft ein neues Verfahren zur Herstellung eines Wittigestersalzes, insbesondere eines (3-Alkoxycarbonyl-2-butenyl)triphenylphosphoniumchlorids oder -alkylsulfats, das jeweils als wichtiger C₅-Baustein für die Herstellung von verschiedenen Polyencarbonsäureestern auf dem Gebiet der Carotinoidchemie bekannt ist.

Das Phosphoniumsalz 3-Aethoxycarbonyl-2-butenyltriphenylphosphoniumchlorid kann bekanntlich aus γ-Chlor-tiglinsäureäthylester durch Umsetzung mit Triphenylphosphin in Toluol bei etwa 110°C leicht hergestellt werden [Nürrenbach et al., Liebigs Ann. Chem. 1977, 1146-1159]. Der γ-Chlor-tiglinsäureäthylester selber und weitere γ-Halogentiglinsäureniederalkylester lassen sich nach verschiedenen bekannten Methoden herstellen, beispielsweise:
- aus dem Tiglinsäurealkylester durch Ziegler-Bromierung (mittels N-Bromsuccinimid in Tetrachlorkohlenstoff) [siehe Inhoffen et al., Liebigs Ann. Chem. 580, 1 (1953); Inhoffen et al., Liebigs Ann. Chem. 580, 7 (1953); sowie Korte et al., Chem. Ber. 89, 2675 (1956)]. Dreiding et al. [Helv. Chim. Acta 53, 383 (1970); siehe auch Cozzi et al., Tetr. Lett. 31, 5661 (1990)] konnten jedoch später zeigen, dass diese Bromierung nicht regioselektiv abläuft und immer ein Gemisch der beiden Isomeren γ-Brom-tiglinsäurealkylester (gewünscht) und 2-Alkoxycarbonyl-1-brom-2-buten (Nebenprodukt) ergibt, welche sich schwer voneinander abtrennen lassen;
- aus dem Brenztraubensäurealkylester durch Umsetzung mit Vinylmagnesiumchlorid (oder mit Brommagnesiumacetylid und nachfolgende partielle Hydrierung), gefolgt von einer Allylumlagerung mit Thionylchlorid (Nürrenbach et al., ibd.) oder einem Phosphortrihalogenid [Kitahara et al., Tetrahedron 44, 4713 (1988)]. Diese Methoden sind allerdings aufwendig und wirtschaftlich uninteressant;
- mittels einer Wittig-Reaktion des Triphenyl(Ph)phosphorans Ph₃P = C(CH₃)COOC₂H₅ mit Chloracetaldehyd [Albertson et al., J. Med. Chem. 20, 602 (1977) sowie Stotter et al., Tetr. Lett. 1975, 1679]. Diese Methode ist ebenfalls aufwendig und wirtschftlich uninteressant;
- ausgehend von 2-Methyl-3-butennitril durch Aethanolyse, Halogenierung und anschliessende Dehydrohalogenierung (Deutsche Offenlegungschrift 3.244.273 sowie US-Patentschrift 4.937.308). Auch diese Methode erweist sich als relativ aufwendig und zudem ungenügend ergiebig; und
- durch Umsetzung von Methylvinylketon mit Blausäure, anschliessende Ueberführung des so erhaltenen Nitrils in einen Ester (Pinner-Reaktion) und Allylumlagerung mit Thionylchlorid oder einem anderen Halogenierungsmittel (Deutsche Offenlegungsschrift 2.852.343 und Nürrenbach et al., ibd). Nachteilig bei dieser Methode ist unter anderem die Verwendung der hochgiftigen Reagenzien Blausäure, Thionylchlorid und Methylvinylketon.

Ziel der vorliegenden Erfindung ist es, ausgehend von einem leicht zugänglichen Ausgangsmaterial und unter möglichst weitgehender Vermeidung der obenerwähnten Nachteile des Standes der Technik ein (3-Alkoxycarbonyl-2-butenyl)triphenylphosphoniumchlorid und das entsprechende Alkylsulfatsalz herzustellen, und zwar auf ökonomische Weise, u.a. durch möglichst wenige Verfahrensschritte.

Das erfindungsgemässe Verfahren zur Herstellung eines (3-Alkoxycarbonyl-2-butenyl)triphenylphosphoniumchlorids oder -alkylsulfats der allgemeinen Formel

X⁻(C₆H₅)₃P⁺CH₂CH=C(CH₃)COOR I

worin R eine Alkylgruppe und X⁻ das Chlor- bzw. Alkylsulfat-Ion (Cl- bzw. ROSO₂O-) bedeuten,
ist dadurch gekennzeichnet, dass man 2-Methyl-3-butennitril der Formel

CH₂=CHCH(CH₃)CN II

mittels eines Alkalimetall- oder Erdalkalimetallhypochlorits α-chloriert, und das so erhaltene 2-Chlor-2-methyl-3-butennitril der Formel

CH₂=CHC(Cl)(CH₃)CN III

entweder zuerst einer Alkanolyse mit einem Alkanol ROH, worin R eine Alkylgruppe bedeutet, zum entsprechenden 2-Chlor-2-methyl-3-butensäurealkylester der allgemeinen Formel

CH₂=CHC(Cl)(CH₃)COOR IV

unterwirft und anschliessend diesen mit Triphenylphosphin zum gewünschten (3-Alkoxycarbonyl-2-butenyl)triphenylphosphoniumchlorid der allgemeinen Formel

Cl⁻(C₆H₅)₃P⁺CH₂CH=C(CH₃)COOR I'

umsetzt,
oder zuerst mit Triphenylphosphin zum (3-Cyano-2-butenyl)triphenylphosphoniumchlorid der Formel

Cl⁻(C₆H₅)₃P⁺CH₂CH=C(CH₃)CN V

umsetzt und anschliessend dieses einer Alkanolyse mit dem Alkanol ROH in Gegenwart konzentrierter Schwefelsäure, zum gewünschten (3-Alkoxycarbonyl-2-butenyl)triphenylphosphoniumalkylsulfat der allgemeinen Formel

ROSO₂O⁻(C₆H₅)₃P⁺CH₂CH=C(CH₃)COOR Iʺ

unterwirft.

In der obigen Definition des erfindungsgemässen Verfahrens ist unter dem Ausdruck "Alkylgruppe" (R) insbesondere eine geradkettige oder verzweigte Alkylgruppe zu verstehen, die 1 bis 4 Kohlenstoffatome enthält. Vorzugsweise ist diese Alkylgruppe Methyl oder Aethyl, insbesondere Aethyl. Bei dem im ersten Schritt des Verfahrens verwendeten Alkalimetall- oder Erdalkalimetallhypochlorit handelt es sich insbesondere um das Natrium-, Kalium- bzw. Calciumhypochlorit.

Das ganze erfindungsgemässe Verfahren kann wie folgt schematisch dargestellt werden: 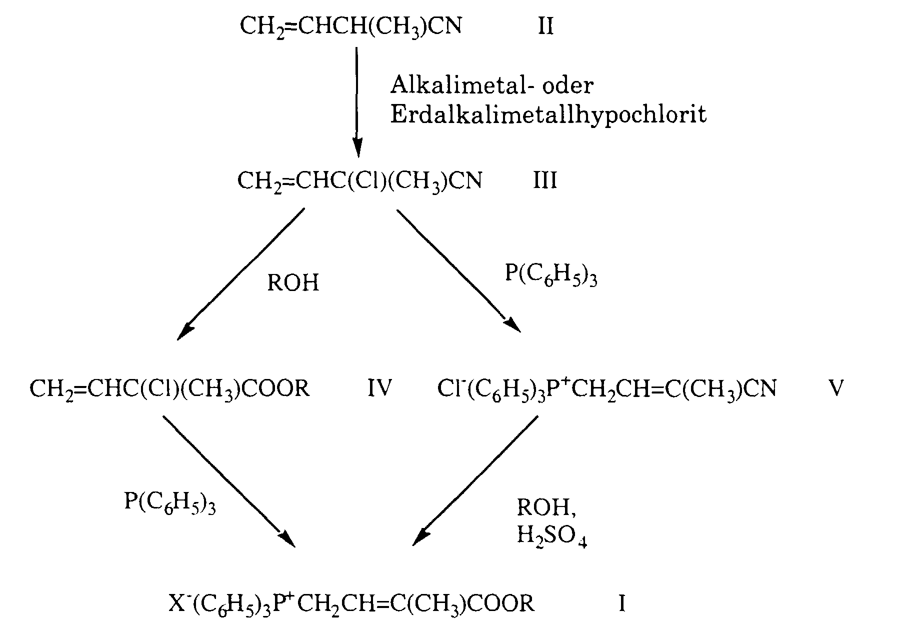 I': X⁻ = Cl⁻ (falls II→III→IV→I': Variante 1)
I": X⁻ = ROSO₂O⁻ (falls II→III→V→I": Variante 2)

Bei dem im erfindungsgemässen Verfahren verwendeten Ausgangsmaterial handelt es sich um das leicht zugängliche und zugleich günstig erhältliche 2-Methyl-3-butennitril, das ein Neben- bzw. Abfallprodukt der Adiponitrilherstellung ist, welches durch direkte Hydrocyanierung von Butadien mit Cyansäure entsteht (siehe beispielsweise die US-Patentschrift 3.850.973).

Das aus diesem Ausgangsmaterial im ersten Schritt des erfindungsgemässen Verfahrens gewonnene 2-Chlor-2-methyl-3-butennitril der Formel III sowie das im nächsten Verfahrensschritt der beiden Varianten jeweils hergestellte Zwischenprodukt, nämlich der 2-Chlor-2-methyl-3-butensäurealkylester der Formel IV und das (3-Cyano-2-butenyl)triphenylphosphoniumchlorid der Formel V, sind hingegen neue Verbindungen. Diese neuen Verbindungen bilden einen weiteren Aspekt der vorliegenden Erfindung

Im Zusammenhang mit der Verbindung der Formel III wird in Chem. Abs. 119, 225949s (1993) [Japanische Patentpublikation (Kokai) 140.113/1993] eine ähnliche Verbindung offenbart, bei der es sich um 2-Chlor-3-butennitril handelt. Im Gegensatz zur bekannten Verbindung weist die Verbindung der Formel III nicht nur ein Chloratom sondern auch noch eine Methylgruppe in 3-Stellung des 3-Butennitrilkerns auf. Ferner wird die bekannte Verbindung zu einem ganz anderen Zweck verwendet als die Verbindung der Formel III, nämlich durch Umsetzung mit Hydrazin(hydrat) zur Herstellung von 5-Amino-3-methylpyrazol.

Die α-Chlorierung des ersten Verfahrensschritts erfolgt zweckmässigerweise unter Einsatz einer wässrigen Lösung des Natrium-, Kalium- oder Calciumhypochlorits und gegebenenfalls eines im wesentlichen mit Wasser nicht mischbaren, aprotischen organischen Lösungsmittels (eines zweiphasigen Lösungsmittelsystems) ohne Katalysator oder in Gegenwart eines Phasentransferkatalysators bei relativ niedrigen Temperaturen. Als wässrige Natriumhypochloritlösung eignet sich insbesondere eine käufliche wässrige "Javellauge", die eine Konzentration von etwa 13 % w/w (Gewichtsprozent) aufweist. Obwohl niedrigere Konzentrationen als etwa 13 Gewichtsprozent verwendet werden können, wird bei diesen eine zunehmende Tendenz zur Isomerisierung des Ausgangsmaterials 2-Methyl-3-butennitril zum gegenüber der Chlorierung inerten 2-Methyl-2-butennitril beobachtet. Die besten Ergebnisse werden mit einer wässrigen Natrium- oder Kaliumhypochloritlösung erzielt, die eine Konzentration an Hypochlorit im Bereich von etwa 10 bis etwa 20 % w/w aufweist. Bei der Verwendung von Calciumhypochlorit als Chlorierungsmittel gilt im allgemeinen ein etwas höherer Konzentrationsbereich, und zwar von etwa 25 bis etwa 35 % w/w. Als organisches Lösungsmittel kommt insbesondere ein aliphatischer Aether, ein niederes, gegebenenfalls halogeniertes Alkan oder ein niederes Cycloalkan, und zwar jeweils mit bis zu 6 Kohlenstoffatomen, oder ein Petroläther in Frage. Bevorzugte organische Lösungsmittel sind Diäthyläther, Diisopropyläther, n-Hexan, Methylenchlorid, Tetrachlorkohlenstoff und Cyclohexan. Die in Betracht kommenden Phasentransferkatalysatoren, die insbesondere quaternäre Ammoniumsalze sind, umfassen unter anderem Aliquat 336, Tetrabutylammoniumbisulfat, Benzyltributylammoniumchlorid, Benzyltriäthylammoniumchlorid, Tetramethylammoniumchlorid und Tetraäthylammoniumbromid, wobei insbesondere die Ammoniumsalze mit niederen Alkylsubstituenten, wie beispielsweise Tetramethylammoniumchlorid und Tetraäthylammoniumbromid, bevorzugt sind. Falls ein Katalysator verwendet wird, setzt man im allgemeinen etwa 2 bis 5 Mol% Katalysator bezogen auf die Menge Ausgangsmaterial ein. Die α-Chlorierung erfolgt vorzugsweise im Temperaturbereich von etwa 0°C bis zur Raumtemperatur, besonders bevorzugt von etwa 5 bis etwa 10°C. Im allgemeinen gilt, dass je höher die Reaktionstemperatur ist, umso schneller ist die Reaktion abgeschlossen. Bei einer Reaktionstemperatur von etwa 5°C beträgt der Umsatz nach fünf Stunden lediglich etwa 55%, während man bei einer Reaktionstemperatur von etwa 10°C nach acht Stunden einen Umsatz von etwa 90% erreicht.

Die gemäss der Variante 1 anschliessende Alkanolyse des 2-Chlor-2-methyl-3-butennitrils der Formel III zum 2-Chlor-2-methyl-3-butensäurealkylester der Formel IV erfolgt zweckmässigerweise entweder durch Zugabe einer Lösung des 2-Chlor-2-methyl-3-butennitrils in einem geeigneten Lösungsmittel zu einer zuvor hergestellten Lösung von Chlorwasserstoff in Alkanol oder durch Einleiten von gasförmigem Chlorwasserstoff in eine Lösung des 2-Chlor-2-methyl-3-butennitrils im Alkanol, das gegebenenfalls im Gemisch mit einem weiteren Lösungsmittel ist. Beide Alternativen erfolgen zweckmässigerweise bei Temperaturen im Bereich von etwa 0°C bis zur Raumtemperatur. Als gegebenenfalls neben dem Alkanol (damit im Gemisch) verwendetes Lösungsmittel dient geeigneterweise ein niederer aliphatischer Aether, z.B. Diäthyl- oder Diisopropyläther, wobei das Volumenverhältnis Alkanol:Aether vorzugsweise von etwa 5:1 bis etwa 1:2, insbesondere etwa 1 : 1, beträgt. Man kann jedoch anstelle eines Aethers beispielsweise ein niederes Alkan, z.B. n-Hexan, oder ein niederes Cycloalkan, z.B. Cyclohexan, das als Lösungsmittel in der Vorstufe verwendet wurde, einsetzen. Weitere Lösungsmittel, z.B. Toluol, können zwar verwendet werden, scheinen jedoch den Umsatz eher negativ zu beeinflussen. Was die Konzentration des Chlorwasserstoffes im Alkanol bzw. im Reaktionsmedium betrifft, ist eine Konzentration nahe der temperaturabhängigen Sättigungsgrenze anzustreben. Im Falle der Zugabe einer Lösung von Chlorwasserstoff in Aethanol beträgt der Chlorwasserstoff-Massenanteil des äthanolischen Chlorwasserstoffes im Temperaturbereich 0 bis 20°C beispielsweise von etwa 47 bis etwa 41%. Im allgemeinen erhöht sich die Ausbeute des 2-Chlor-2-methyl-3-butensäure-alkylesters wesentlich mit steigender Chlorwasserstoff-Konzentration. Darüber hinaus sollte zweckmässigerweise die Temperatur des Reaktionsgemisches zu Beginn der Alkanolyse etwa 10°C nicht übersteigen, um ein unnötiges Ausgasen von Chlorwasserstoff aus der alkanolischen Phase zu begrenzen. Im Laufe der Reaktion kann dann die Temperatur sukzessiv erhöht werden, beispielsweise um etwa 2,5°C/Stunde, und die besten Ausbeuten scheinen bei Tempetaturen von etwa 15°C bis etwa 17,5°C realisiert zu werden.

Im Anschluss an die Reaktion wird das Reaktionsgemisch dieses zweiten Verfahrensschrittes hydrolysiert, und zwar zweckmässigerweise durch Zugabe eines Eis/Wasser-Gemisches bei etwa 0°C zum Reaktionsgemisch, oder umgekehrt. Während der Zugabe fällt aus der Alkanolphase normalerweise kristallines Ammoniumchlorid aus, welches je nach zugegebener Wasser/Eis-Menge wieder vollständig in Lösung geht.

Wie aus der obigen Reaktionsbeschreibung ersichtlich, kann man unter Umständen das Rohprodukt der α-Chlorierungssstufe direkt in der anschliessenden Alkanolyse umsetzen, ohne das im ersten Verfahrensschritt verwendete Lösungsmittel, z.B. Diäthyl- oder Diisopropyläther, n-Hexan oder Cyclohexan, abzutrennen, da das jeweilige Lösungsmittel auch im zweiten Verfahrensschritt verwendbar ist.

Für die letzte Stufe dieser Variante des erfindungsgemässen Verfahrens, nämlich die Ueberführung des 2-Chlor-2-methyl-3-butensäure-alkylesters in das Phosphoniumsalz (3-Alkoxycarbonyl-2-butenyl)triphenylphosphoniumchlorid der Formel I', wurde überraschenderweise gefunden, dass sich die Umsetzung mit Triphenylphosphin in einem organischen Lösungsmittel, geeigneterweise einem Aromaten, z.B. Benzol, Toluol oder einem Xylol, einem Dialkylketon, z.B. Methylisobutylketon, oder einem Alkansäure-alkylester, z.B. Aethyl-n-butyrat oder Aethylisobutyrat, bei erhöhter Temperatur, z.B. im Temperaturbereich von etwa 90 bis etwa 150°C, vorzugsweise von etwa 110 bis etwa 130°C, insbesondere bei der Rückflusstemperatur des Reaktionsgemisches (bei etwa 110°C im Falle der Verwendung von Toluol als Lösungsmittel) durchführen lässt.

Das Rohprodukt des zweiten Verfahrensschrittes, d.h. der 2-Chlor-2-methyl-3-butensäure-alkylester der Formel IV, kann auch ohne vorherige Isolierung im dritten Verfahrensschritt eingesetzt werden, wobei man im Falle der Verwendung eines zweiten Lösungsmittels, z.B. n-Hexan, dieses durch das für die Phosphoniumsalz-Bildung geeignete Lösungsmittel, z.B. ein Aromat, wie Toluol, austauschen soll.Falls hingegen im zweiten Verfahrenschritt das Alkanol als alleiniges Lösungsmittel verwendet worden ist, kann man vorteilhaft mit dem für die Phosphoniumsalz-Bildung vorgesehenen Lösungsmittel den Ester der Formel IV extrahieren und dadurch einen Lösungsmittelaustausch vermeiden.

Falls nach Durchführung des ersten und/oder des zweiten Verfahrensschrittes das jeweilige Produkt der Formel III bzw. IV wunschgemäss zu isolieren und reinigen ist, so kann dies auf an sich bekannte Weise erfolgen.

Die oben beschriebene Sequenz der Reaktionsschritte α-Chlorierung, Alkanolyse und Phosphoniumsalz-Bildung stellt die Variante 1 des erfindungsgemässen Verfahrens dar. Bei der Variante 2 werden im Prinzip die beiden letzten Verfahrensschritte in umgekehrter Reihenfolge durchgeführt, wobei allerdings unter Einwirkung der Schwefelsäure in der Alkanolyse statt des Phosphoniumchlorids das entsprechende Alkylsulfatsalz erhalten wird.

Die bei der Variante 2 des erfindungsgemässen Verfahrens involvierte Umsetzung des 2-Chlor-2-methyl-3-butennitrils der Formel III mit Triphenylphosphin kann analog der bereits beschriebenen Phosphoniumsalz-Bildung durchgeführt werden, d.h. zweckmässigerweise unter Verwendung eines Aromaten, z.B. Benzol, Toluol oder eines Xylols, eines Dialkylketons oder eines Alkansäure-alkylesters, als Lösungsmittel, und bei erhöhter Temperatur, wie beispielsweise im Temperaturbereich von etwa 70°C bis etwa 130°C, vorzugsweise bei der Rückflusstemperatur des Reaktionsgemisches.

Die anschliessende Alkanolyse des so hergestellten (3-Cyano-2-butenyl)triphenylphosphoniumchlorids der Formel V muss im Gegensatz zu der Alkanolyse der Variante 1 in Gegenwart von Schwefelsäure anstelle des Chlorwasserstoffs durchgeführt werden, um zum (3-Alkoxycarbonyl-2-butenyl)triphenylphosphonium-alkylsulfat der Formel I" zu gelangen. Die Umsetzung, deren Erfolg als überraschend anzusehen ist, gelingt unter verhältnismässig drastischen Reaktionsbedingungen und erfolgt zweckmässigerweise in einem Gemisch des Alkanols und konzentrierter, vorzugsweise 95 bis 97%iger, Schwefelsäure unter erhöhter Temperatur, vorzugsweise bei der Rückflusstemperatur des Reaktionsgemisches. Das molare Verhältnis von Alkanol zu Schwefelsäure liegt zweckmässigerweise im Bereich von etwa 1:1 bis etwa 3:1, vorzugsweise von etwa 2:1.

Falls nach Durchführung des ersten Verfahrensschrittes der Variante 2 das Produkt der Formel III wunschgemäss zu isolieren und reinigen ist, so kann dies auch in diesem Fall auf an sich bekannte Weise erfolgen. Analog der Variante 1 kann man allerdings das Rohprodukt der α-Chlorierungsstufe direkt mit Triphenylphosphin umsetzen, ohne das im ersten Verfahrensschritt verwendeten Lösungsmittel, z.B. Toluol, abzutrennen.

Ein weiterer Aspekt des erfindungsgemässen Verfahrens besteht darin, das man vom 2-Methyl-3-butennitril zum (3-Cyano-2-butenyl)triphenylphosphoniumchlorid über das 3,4-Dichlor-2-methylbutannitril [der Formel ClCH₂CH(Cl)CH(CH₃)CN], gefolgt vom 4-Chlor-2-methyl-2-butennitril [der Formel ClCH₂CH=C(CH₃)CN] gelangt. Das diesbezügliche Verfahren besteht darin, dass man 2-Methyl-3-butennitril mit elementarem Chlor zum 3,4-Dichlor-2-methylbutannitril chloriert, dieses unter Verwendung einer Base dehydrochloriert und das so erhaltene 4-Chlor-2-methyl-2-butennitril mit Triphenylphosphin zum gewünschten (3-Cyano-2-butenyl)triphenylphosphoniumchlorid umsetzt. Das 3,4-Dichlor-2-methylbutannitril ist eine neue Verbindung, das 4-Chlor-2-methyl-2-butennitril hingegen eine bekannte [siehe beispielsweise Lugtenburg et al., Recl. Trav. Chim. Pays-Bas 109, 378 (1990)].

Die Chlorierung des 2-Methyl-3-butennitrils wird zweckmässigerweise in einem niederen, gegebenenfalls halogenierten Kohlenwasserstoff als Lösungsmittel bei niedrigen Temperaturen, insbesondere unterhalb von 0°C, und gegebenenfalls in Gegenwart einer Base durchgeführt. Als Lösungsmittel kommen insbesondere gegebenenfalls halogenierte, vorzugsweise chlorierte, Alkane mit bis zu 6 Kohlenstoffatomen, wie beispielsweise n-Pentan, n-Hexan, Methylenchlorid und Tetrachlorkohlenstoff, in Betracht. Der Temperaturbereich, in dem die Umsetzung erfolgt, ist geeigneterweise von etwa -80°C bis etwa +30°C, wobei vorzugsweise bei etwa -20°C bis etwa +20°C umgesetzt wird. Falls eine Base verwendet wird, ist diese vorzugsweise Pyridin, das zudem als Lösungsmittel dienen kann. Es hat sich als vorteilhaft erwiesen, das 2-Methyl-3-butennitril, gegebenenfalls in Lösung in einem Teil des verwendeten Lösungsmittels, in eine Lösung des Chlors im restlichen Lösungsmittel zuzutropfen. Man kann aber die inverse Zugabe (Zutropfen der Chlorlösung) durchführen, was zu ähnlichen Ergebnissen führt, oder das Chlor unmittelbar in die Lösung des 2-Methyl-3-butennitrils einleiten.

Die anschliessende Dehydrochlorierung erfolgt zweckmässigerweise unter Verwendung eines Alkalialkoholats oder Alkalihydroxids in einem niederen Alkohol und/oder in Wasser. Man kann allerdings als Alternative einen gegebenenfalls halogenierten niederen Kohlenwasserstoff, insbesondere einen mit bis zu 6 Kohlenstoffatomen, wie beispielsweise n-Pentan, n-Hexan oder Methylenchlorid, oder ein Aromat, z.B. Toluol, als Lösungsmittel verwenden. Je nach verwendetem Lösungsmittel wird zudem ein Phasentransferkatalysator benötigt, wie etwa im Falle der Verwendung eines halogenierten Kohlenwasserstoffs oder eines Aromaten. Bei der Verwendung von n-Pentan oder n-Hexan erübrigt sich hingegen der Phasentransferkatalysator. Beispiele der verwendbaren Phasentransferkatalysatoren sind diejenigen, welche oben im Zusammenhang mit der α-Chlorierung von 2-Methyl-3-butennitril genannt sind. Besonders bevorzugt ist Tetrabutylammoniumchlorid. Die Dehydrochlorierung erfolgt geeigneterweise bei Temperaturen zwischen etwa -20°C und etwa +40°C, vorzugsweise bei Raumtemperatur.

Der letzte Verfahrensschritt in dieser Sequenz, nämlich die Umsetzung von 4-Chlor-2-methyl-2-butennitril mit Triphenylphosphin, erfolgt zweckmässigerweise in einem niederen Alkanol, z.B. Isopropanol, als Lösungsmittel und bei erhöhter Temperatur, vorzugsweise bei der Rückflusstemperatur des Reaktionsgemisches.

Das erfindungsgemäss hergestellte (3-Alkoxycarbonyl-2-butenyl)triphenylphosphoniumchlorid oder -alkylsulfat kann zur Herstellung von verschiedenen Polyencarbonsäureestern verwendet werden, und zwar durch Umsetzung mit entsprechenden Carbonylverbindungen nach Wittig. Beispiele verschiedener Endprodukte (Polyencarbonsäure-alkylester) sind β-Apo-8'-carotinsäure-alkylester [Guex et al., US-Patentschrift 3.113.961], β-Apo-4'-carotinsäure-alkylester [Neurosporaxanthin-alkylester; Isler et al., Helv. Chim. Acta 42, 864 (1959)] und Crocetin-alkylester [Buchta et al., Chem. Ber. 93, 1349 (1960)].

Die Erfindung wird anhand der nachfolgenden Beispiele veranschaulicht.

### Beispiel 1

### Herstellung von 2-Chlor-2-methyl-3-butennitril

10 g (0,1 Mol) 2-Methyl-3-butennitril [etwa 85%ig rein gemäss Gaschromatographie (GC)] werden zu einem Gemisch von 100 ml Methylenchlorid/50 ml Wasser gegeben und bei Raumtemperatur mit 100 ml 10%iger Javellauge (etwa 0,15 Mol) versetzt, gefolgt von 3 g (10 Mol %) Tetrabutylammoniumhydrogensulfat. Das Reaktionsgemisch wird 16 Stunden bei Raumtemperatur gerührt und danach einer konventionellen Aufarbeitung unterworfen. Das isolierte Rohprodukt besteht gemäss GC zu 56 % aus 2-Chlor-2-methyl-3-butennitril und zu 19 % aus 2-Methyl-2-butennitril.

Man destilliert das Rohprodukt bei 67°C/80 mm Hg (10,67 kPa) an einer kleinen Man destilliert das Rohprodukt bei 67°C/80 mm Hg an einer kleinen Vigreux-Kolonne und chromatographiert die erste Fraktion (5,4 g) an 250 g Kieselgel (Ø 0,04 - 0,063) unter Verwendung von n-Hexan/Aethylacetat (19 : 1) als Eluierungsmittel, was nach anschliessender Destillation im Kugelrohr bei ca. 50°C/15 mm Hg (2 kPa) 900 mg reines farbloses 2-Chlor-2-methyl-3-butennitril ergibt. Gemäss GC beträgt der Reinheitsgrad ca. 99 %.

### Analytische Daten

- ¹H-NMR (250 MHz, CDCl₃):: 2,01 (s, 3H), 5,38 (d, J=10 Hz, 1H), 5,70 (d, J=16 Hz, 1H), 5,96 (2d, J₁=16, J₂=10, 1H) ppm.
- IR (Film):: 2240w, 1640w, 1412s, 1050s, 943s, 803s;
- MS:: 115 (M⁺, 2), 100(14), 80(100), 53(90).

| | | | | | |
|---|---|---|---|---|---|
| Mikroanalyse: | Ber.: | C 51,97 % | H 5,23 % | N 12,12 % | Cl 30,68 % |
| | Gef.: | C 52,10 % | H 5,50 % | N 11,83 % | Cl 30,75 % |

### Beispiel 2

### Herstellung von 2-Chlor-2-methyl-3-butennitril (ohne Verwendung eines Katalysators)

In einem mit Kühler, Thermometer, Thermostat sowie mechanischem Rührer ausgestatteten 1 l-Doppelmantelkolben werden 102 g (1,1 Mol) 2-Methyl-3-butennitril (etwa 84%ig rein gemäss GC) in 500 ml n-Hexan vorgelegt, gerührt und auf 10°C abgekühlt. Zur resultierenden Lösung werden dann innert etwa 20 Minuten 700 g 13%ige Javellauge portionenweise zugegeben, und das Ganze wird anschliessend 7 Stunden bei 10°C gerührt (Eisbadkühlung). Danach wird das Gemisch sowie die Spülungen mit 100 ml n-Hexan in einen Scheidetrichter übertragen und die organische Phase abgetrennt. Die vereinigten organischen Phasen werden schliesslich mit 100 ml deionisiertem Wasser gewaschen.

Die so erhaltene Hexanlösung enthält etwa 20-22 Gewichtsprozent 2-Chlor-2-methyl-3-butennitril (Ausbeute etwa 89-92% gemäss GC) und kann direkt in den nächsten Verfahrenschritt (die Alkanolyse) eingesetzt werden.

### Beispiel 3

### Herstellung von 2-Chloro-2-methyl-3-butensäure-äthylester aus 2-Methyl-3-butennitril über 2-Chlor-2-methvl-3-butennitril (ohne Isolierung und Reinigung des Zwischenproduktes - 1. Durchprozessvariante)

In einem mit mechanischem Rührer und Thermometer ausgestatteten 1,5 l-Sulfierkolben werden 49,0 g (0,5 Mol) 2-Methyl-3-butennitril (ca. 83%ig rein) in 250 ml n-Hexan vorgelegt, und das Gemisch wird auf ca. 5°C abgekühlt (Eisbad). Dazu werden auf einmal unter Rühren 400 g (0,7 Mol, 1,4 Aeq.) frische, auf ca. 5°C vorgekühlte 13%ige Javellauge zugegeben, gefolgt von 2,8 g (26 mMol, ca. 5 Mol %) Tetramethylammoniumchlorid. Dann wird ca. 16 Stunden bei ca. +5°C (Eisbad) und anderthalb Stunden bei Raumtemperatur gerührt. Danach wird die Hexanphase abgetrennt und der Kolben mit 50 ml n-Hexan gespült, welches zugleich benutzt wird, um die Wasserphase nochmals zu extrahieren. Die vereinigte organische Phase wird nun mit 50 ml Wasser gewaschen. Man tropft die resultierende Lösung des rohen 2-Chlor-2-methyl-3-butennitrils in n-Hexan (ca. 300 ml) nun unter Rühren bei 5°C zu einer vorher bei 5°C hergestellten Lösung von 130 g (3,5 Mol, 7 Aeq.) gasförmigem Chlorwasserstoff in 200 ml Aethanol in einem mit mechanischem Rührer, Thermometer und Gaseinleitungsrohr versehenen 1,5 l Sulfierkolben innert 30 Minuten. Das Gemisch wird ca. 16 Stunden bei 0-5°C und ca. 2-3 Stunden bei Raumtemperatur gerührt. Dann wird das Reaktionsgemisch auf 300 g Eis geleert und eine Stunde gerührt. Man trennt die wässrige Phase ab und extrahiert zweimal mit 150 ml, insgesamt 300 ml, n-Hexan. Die vereinigte organische Phase wird mit 100 ml gesättigter Natriumbicarbonat-Lösung gewaschen, mit 30 g wasserfreiem Natriumsulfat getrocknet und abfiltriert, und das Lösungsmittel (n-Hexan) wird über eine Raschig-Kolonne (Durchmesser: 2,5 cm; Länge: 30 cm) bei Normaldruck abdestilliert. Den Rückstand destilliert man an einer Vigreux-Kolonne (NS 29,5; Durchmesser: 2,5 cm; Länge: 20 cm) bei 12 mbar (1,2 kPa). Dies ergibt bei einem Siedepunkt von 53-57°C/12 mbar (1,2 kPa) 64,6 g (77,3 % bezogen auf 2-Methyl-3-butennitril) 2-Chlor-2-methyl-3-butensäure-äthylester als wasserklare Flüssigkeit mit einer Reinheit gemäss GC von 97,2 % (Retentionszeit: 7,8 Minuten).

### Analytische Daten

- ¹H-NMR (250 MHz, CDCl₃):: 1,31 (t, J=7 Hz, 3H), 1,86 (s, 3H), 4,25 (q, J=7 Hz, 2H), 5,27 (d, J=12 Hz, 1H), 5,43 (d, J=16 Hz, 1H), 6,21 (2d, J₁=16 Hz, J₂=10 Hz, 1H) ppm.
- IR (Film):: 1740s, 1640w, 1265s, 1125s, 991m, 933m;
- MS:: 127 (M-Cl, 5),99(15), 91(32), 89(100), 53(54).

| | | | | |
|---|---|---|---|---|
| Mikroanalyse: | Ber.: | C 51,70 % | H 6,82 % | Cl 21,80 % |
| | Gef.: | C 51,68 % | H 6,96 % | Cl 21,90 %. |

### Beispiel 4

### Herstellung von 2-Chlor-2-methvl-3-butensäure-äthylester aus 2-Methyl-3-butennitril über 2-Chlor-2-methyl-3-butennitril (ohne Isolierung und Reinigung des Zwischenproduktes - 2, Durchprozessvariante)

Diese Variante wird wie die obige 1. Variante durchgeführt, ausser dass Cyclohexan anstelle von n-Hexan als Lösungs- und Extraktionsmittel verwendet wird.

Bei analoger Umsetzung von 49,0 g (0,5 Mol) 2-Methyl-3-butennitril (ca. 83%ig rein) werden am Schluss der Reaktionsfolge 63,0 g (75,3 % bezogen auf 2-Methyl-3-butennitril) 2-Chlor-2-methyl-3-butensäure-äthylester als wasserklare, farblose Flüssigkeit erhalten (Sdp.: 55-60°C/15 mbar (1,5 kPa), mit einer Reinheit gemäss GC von 97,1 %).

### Beispiel 5

### Herstellung von 2-Chlor-2-methyl-3-butensäure-äthylester aus 2-Methyl-3-butennitril über 2-Chlor-2-methyl-3-butennitril (ohne Isolierung und Reinigung des Zwischenproduktes - 3, Durchprozessvariante)

In einem mit mechanischem Rührer und Thermostat ausgestatteten 1,5 l -Vierhalssulfierkolben werden 48 g (0,5 Mol) 2-Methyl-3-butennitril (ca. 84,5%ig rein) in 250 ml tiefsiedendem Petroläther vorgelegt. Dazu werden bei 0°C unter Rühren eine Suspension von 105 g (0,5 Mol) Calciumhypochlorit in 250 ml Wasser, gefolgt von 5 g (24 mMol, 2,4 Mol %) Tetraäthylammoniumbromid zugegeben. Man rührt das Reaktionsgemisch bei 0°C 2 Stunden und bei Raumtemperatur weitere 16 Stunden, und unterwirft eine Probe der GC-Analyse. Dies zeigt, dass das Rohprodukt u.a. zu 41 % aus 2-Chlor-2-methyl-3-butennitril und zu 37 % aus Ausgangsmaterial besteht.

Dann werden nochmals 50 g (0,25 Mol) Calciumhypochlorit in 100 ml Wasser zugegeben, und das Reaktionsgemisch wird 24 Stunden bei Raumtemperatur und 2 Stunden bei 30°C gerührt, was zu einem Produkt mit 74,3 % 2-Chlor-2-methyl-3-butennitril gemäss GC führt. Man filtriert die Suspension und wäscht das Filtergut mit 150 ml tiefsiedendem Petroläther. Vom zweiphasigen Filtrat wird die organische Phase abgetrennt, und diese wird mit 50 ml Wasser gewaschen, über wasserfreiem Natriumsulfat getrocknet und filtriert. Das Lösungsmittel wird nun über eine Raschig-Kolonne (Durchmesser: 2,5 cm, Länge: 30 cm) bei Normaldruck bis ca. 250 g Sumpfgewicht abdestilliert. Diese Lösung wird nun mit 200 ml Diäthyläther verdünnt und unter Rühren bei 5°C zu einer vorher hergestellten Lösung von 200 g (5,5 Mol, 11 Aeq.) gasförmigem Chlorwasserstoff in 200 ml absolutem Aethanol innert ca. 30 Minuten getropft. Man rührt das Gemisch ca. 16 Stunden bei 5°C, dann eine Stunde bei Raumtemperatur. Eine Probe des Gemisches weist einen Gehalt an 2-Chlor-2-methyl-3-butensäure-äthylester von 84,6 % auf. Zur Aufarbeitung wird auf 500 g Eis geleert, eine Stunde bei Raumtemperatur nachgerührt und mit 100 g festem Kochsalz versetzt. Man trennt die organische Phase ab und extrahiert die Wasserphase noch dreimal mit je 250 ml, insgesamt 750 ml, Diäthyläther. Die vereinigten Aetherphasen werden einmal mit 100 ml gesättigter Natriumbicarbonat-Lösung gewaschen, über wasserfreiem Natriumsulfat getrocknet, filtriert und eingeengt. Die anschliessende Destillation an einer Vigreux-Kolonne (Durchmesser 2,5 cm; Länge 30 cm) ergibt bei 54-58°C/14 mbar (1,4 kPa) 51,0 g (60 %) 2-Chlor-2-methyl-3-butensäure-äthylester als farblose Flüssigkeit mit einer Reinheit gemäss GC von 95,1 %.

### Beispiel 6

### Herstellung von (3-Aethoxycarbonyl-2-butenyl)triphenylphosphoniumchlorid aus 2-Chlor-2-methyl-3-butensäure-äthylester

In einem mit mechanischem Rührer, Thermometer und Kühler versehenen 2,5 l-Vierhalssulfierkolben werden 84,0 g (0,48 Mol, GC-korrigiert) 2-Chlor-2-methyl-3-butensäure-äthylester (93%ig rein gemäss GC) und 144 g (0,55 Mol) Triphenylphosphin in 1 l Toluol gelöst und 48 Stunden am Rückfluss erhitzt (beim Aufheizen bei ca. 100°C impft man mit bereits hergestelltem Phosphoniumsalz). Nach Abkühlung auf Raumtemperatur wird der Kristallbrei filtriert, dreimal mit je 300 ml Toluol, insgesamt 900 ml, Toluol, gewaschen und im Trockenschrank 20 Stunden bei 50°C unter Wasserstrahlvakuum getrocknet. Dies ergibt 187,8 g (88 %) (3-Aethoxycarbonyl-2-butenyl)triphenylphosphoniumchlorid als leicht beiges Pulver mit einem Smp. von 191-192°C und einer Reinheit gemäss HPLC von 95,7 %.

### Beispiel 7

### Herstellung von (3-Aethoxycarbonyl-2-butenyl)triphenylphosphoniumchlorid aus 2-Methyl-3-butennitril über 2-Chlor-2-methyl-3-butennitril und 2-Chlor-2-methyl-3-butensäure-äthylester (ohne Isolierung und Reinigung der beiden Zwischenprodukte - 4, Durchprozessvariante)

In einem mit mechanischem Rührer und Thermometer ausgestatteten 1,5 l-Vierhalssulfierkolben werden 51 g (0,5 Mol) 2-Methyl-3-butennitril (ca. 80%ig rein) in 250 ml n-Hexan vorgelegt, das Gemisch wird auf ca. 5°C abgekühlt (Kryostat). Dann werden bei 5°C innert ca. 20 Minuten unter Rühren 350 g (0,63 Mol, 1,25 Aeq.) frische 13%ige Javellauge zugetropft und anschliessend 1,4 g (13 mMol, 2,5 Mol %) Tetramethylammoniumchlorid zugegeben. Nach 16-stündigem Rühren bei 5°C (Kryostat) wird die Hexanphase abgetrennt und der Kolben mit 50 ml n-Hexan gespült, welches zugleich benutzt wird, um die Wasserphase nochmals zu extrahieren. Die vereinigte organische Phase wird schliesslich mit 50 ml Wasser gewaschen.

Diese Lösung des rohen 2-Chlor-2-methyl-3-butennitrils in n-Hexan (ungetrocknet, ca. 300 ml) wird nun innert ca. 30 Minuten unter Rühren zu einer vorher hergestellten Lösung von 100 g (2,75 Mol, 5,5 Aeq.) gasförmigem Chlorwasserstoff in 200 ml absolutem Aethanol bei 10°C zugetropft, und zwar in einem mit mechanischen Rührer, Thermometer und Gaseinleitungsrohr versehenen 1,5 l Vierhalssulfierkolben. Das resultierende Gemisch wird 16 Stunden bei 15°C (Kryostat) gerührt und danach abgekühlt. Zur Lösung gibt man 300 g Eis zu, und die Lösung wird dann ca. 30 Minuten bei Raumtemperatur gerührt. Die wässrige Phase wird abgetrennt und zweimal mit je 75 ml, insgesamt 150 ml, n-Hexan extrahiert. Man wäscht die vereinigte organische Phase mit 75 ml gesättigter Natriumbicarbonat-Lösung und destilliert anschliessend (ohne Trocknung) das n-Hexan durch eine Raschig-Kolonne (30 x 2,5 cm) bei Normaldruck ab. Dies ergibt einen gelben Rückstand (92,6 g), welchen man in 1 l Toluol löst. Zur Lösung in einem 2,5 l Vierhalssulfierkolben, der mit mechanischem Rührer und Thermometer versehen ist, gibt man 131 g (0,5 Mol) Triphenylphosphin zu. Das Gemisch wird anschliessend 40 Stunden am Rückfluss erhitzt (nach ca. 30 Minuten impft man mit bereits hergestelltem Phosphoniumsalz). Der angefallene Kristallbrei wird auf 20°C abgekühlt, abgenutscht, dreimal mit je 250 ml, insgesamt 750 ml, Toluol gewaschen und im Vakuumtrockenschrank 16 Stunden bei ca. 50°C getrocknet. Dies ergibt 162,8 g (74,4 % bezogen auf 2-Methyl-3-butennitril) beiges (3-Aethoxycarbonyl-2-butenyl)triphenylphosphoniumchlorid mit Smp. 191°C und einer Reinheit gemäss HPLC von 97,1%.

### Beispiel 8

### Herstellung von (3-Cyano-2-butenyl)triphenylphosphoniumchlorid aus 2-Methyl-3-butennitril über 2-Chlor-2-methyl-3-butennitril (ohne Isolierung und Reinigung des Zwischenproduktes - "Durchprozess")

49 g (0,5 Mol) 2-Methyl-3-butennitril (84%ig rein gemäss GC) werden in 250 ml Toluol vorgelegt, und das Gemisch wird auf 5°C gekühlt. Dazu werden innert 30 Minuten 350 g 13%ige Javellauge (0,63 Mol) zugetropft, und anschliessend 1,4 g Tetramethylammoniumchlorid zugegeben. Das resultierende Zweiphasengemisch wird 16 Stunden bei 0-5°C und noch 3 Stunden bei Raumtemperatur gerührt. Dann wird die wässrige Phase abgetrennt und nochmals mit 100 ml Toluol extrahiert. Die vereinigten organischen Phasen werden mit 50 ml Wasser gewaschen, über wasserfreiem Natriumsulfat getrocknet und filtriert. Dies ergibt 500 g einer Lösung von rohem 2-Chlor-2-methyl-3-butennitril in Toluol.

Zu 250 g dieser Toluollösung werden zusätzlich 200 ml Toluol gegeben. Nach Zugabe von 70 g (ca. 0,26 Mol) Triphenylphosphin wird die gelbliche Lösung auf 90°C erwärmt (nach 10 Minuten beginnt sich ein Niederschlag zu bilden). Dann wird 16 Stunden bei 100°C weitergerührt. Man kühlt anschliessend das Gemisch auf 5°C, filtriert den Niederschlag ab, wäscht ihn dreimal mit je 150 ml, insgesamt 450 ml, Toluol und trocknet ihn im Wasserstrahlvakuum bei 50°C während 4 Stunden. Dies ergibt 79,0 g (80,3 %, korrigiert) (E/Z)-(3-Cyano-2-butenyl)triphenylphosphoniumchlorid als fast weisses Pulver mit Smp. 277-278°C; HPLC: 96%ig rein; (E/Z)-Gemisch (E/Z-Verhältnis ca. 1 : 1).

| | | | | | |
|---|---|---|---|---|---|
| Mikroanalyse: | Ber.: | C 73,11 % | H 5,60 % | N 3,71 % | Cl 9,38 % |
| | Gef.: | C 72,99 % | H 5,49 % | N 3,43 % | Cl 9,66 % |

### Beispiel 9

### Herstellung von (3-Aethoxycarbonyl-2-butenyl)triphenylphosphoniumäthylsulfat aus (3-Cyano-2-butenyl)triphenylphosphoniumchlorid

In einem mit mechanischem Rührer, Kühler, Thermometer und Tropftrichter versehenen 350 ml -Vierhalssulfierkolben werden unter Argon 20,0 g (0,051 Mol) (E/Z)-(3-Cyano-2-butenyl)triphenylphosphoniumchlorid [E/Z-Verhältnis ca. 5 : 1 gemäss ¹H-NMR(CDCl₃); gemäss HPLC ca. 97,4 %ig rein] und 54 ml/42,7 g (0,92 Mol) Aethanol vorgelegt. Zu der resultierenden Suspension wird unter Aceton/Eiskühlung, Argon und Rühren (500 U/Minute), innert 20 Minuten 26 ml/47,5 g (0,46 Mol) konzentrierte Schwefelsäure (95 - 97%ig rein, gemäss GC) so zugetropft, dass die Temperatur bei 3° bis 5°C gehalten wird. Die viskose, farblose Lösung wird 20 Stunden am Rückfluss gerührt (Oelbadtemperatur: 100°C). Die Innentemperatur von anfänglich 92°C senkt allmählich auf 83°C. Nach Abkühlung im Eisbad wird das Reaktionsgemisch abgenutscht und das Filtergut dreimal mit je 50 ml, insgesamt 150 ml, Methylenchlorid gewaschen. Im farblosen Niederschlag (mineralischem Salz) werden Ammonium-, Chlorid- und Sulfat-Ionen nachgewiesen. Das Filtrat wird fünfmal mit je 40 ml, insgesamt 200 ml, Wasser neutral gewaschen. Die organische Phase wird über wasserfreiem Natriumsulfat getrocknet, filtriert und in einem 1 l Rundkolben bei 35°C unter Wasserstrahlvakuum im Rotationsverdampfer eingedampft. Dies ergibt 26,9 g rohes (E)-(3-Aethoxycarbonyl-2-butenyl)triphenylphosphonium-äthylsulfat als hellgelben Schaum, der gemäss HPLC eine Reinheit von 88,4 % aufweist.

Zur Reinigung des Produktes wird der Schaum (26,9 g) in 60 ml heissem Aceton gelöst. Aus der Lösung, die vorerst im Eisbad abgekühlt wird, kristallisiert das Produkt. Zur Vollständigkeit der Kristallisation werden nach 30 Minuten 150 ml Diäthyläther zugegeben, und die Lösung wird noch eine Stunde im Eisbad gerührt. Das resultierende Kristallisat wird abgenutscht, zweimal mit je 25 ml, insgesamt 50 ml, Diäthyläther gewaschen und über Phosphorpentoxid bei 22 mbar (2,2 kPa) während 16 Stunden getrocknet. Dies ergibt 22,8 g (E)-(3-Aethoxycarbonyl-2-butenyl)triphenylphosphoniumäthylsulfat [82 % bezogen auf (3-Cyano-2-butenyl)triphenylphosphoniumchlorid] als farbloses Pulver, Smp. 121-122°C; HPLC: 94,4%ig rein; Dünnschichtchromatographie: R_{f} = 0,45 mit Laufmittel Aethylacetat: Aceton : Ameisensäure 8 : 1: 1 und Jod als Entwickler.

Eine Umkristallisation aus 60 ml Aceton und 150 ml Diäthyläther, wie oben beschrieben, ergibt 21,9 g Produkt (79 % bezogen auf das Ausgangsmaterial) als farbloses Pulver, Smp. 123-125°C; HPLC: 95,52%ig rein.

### Beispiel 10

### Herstellung von (l.u)-3,4-Dichlor-2-methyl-butannitril aus 2-Methyl-3-butennitril

In einem mit mechanischem Rührer, Thermometer und Gaseinleitungsrohr versehenen 200 ml -Vierhalssulfierkolben werden unter Argon eine Lösung von 39,6 g (0,4 Mol, 1 Aeq.) 2-Methyl-3-butennitril in 5,6 g (0,071 Mol, 0,18 Aeq.) Pyridin (99,8%ig rein gemäss GC) vorgelegt. Unter Kühlung (Aceton/Eisbad) werden unter Argon innert 34 Minuten bei einer Durchflussmenge von 1 g/Minute (Einstellung mit Rotameter) ca. 34 g (0,48 Mol, 1,2 Aeq.) Chlor (99,9%ig rein gemäss GC, über konzentrierte Schwefelsäure getrocknet) eingeleitet. Die aufgenommene Chlormenge wird durch Abwägen des Kolbens kontrolliert. Dabei steigt die Temperatur von ca. -10°C auf 20°C. Zuerst bildet sich ein farbloser Niederschlag (Pyridiniumhydrochlorid), der sich allmählich in einen schmierigen Klumpen umwandelt. Das Reaktionsgemisch wird auf 20 ml Wasser gegossen. Der Reaktionskolben wird zweimal mit je 25 ml, insgesamt 50 ml, Wasser gespült. Man wäscht die organische Phase viermal mit je 25 ml, insgesamt 100 ml, Wasser, trocknet sie über wasserfreiem Natriumsulfat und filtriert sie. Jede wässrige Phase wird mit 25 ml, insgesamt (4 Waschphasen) 100 ml, n-Pentan extrahiert. Die Pentan-Phasen werden zweimal mit je 25 ml, insgesamt 50 ml, Wasser neutral gewaschen, über wasserfreiem Natriumsulfat getrocknet und filtriert. Die gesamte organische Phase wird bei ca. 15°C unter Wasserstrahlvakuum eingedampft (Rotationsverdampfer versehen mit einem mit Trockeneis/Aceton gefüllten Dewar-Kühler). Dies ergibt 60 g rohes (l,u)-3,4-Dichlor-2-methyl-butannitril als farbloses Oel: GC (1 + u) = 91,9%; Diastereomerenverhältnis (l) : (u) ca. 1: 1.

Eine Destillation über eine Vigreux-Kolonne (5 cm) ergibt 52,3 g (74%) (l,u)-Produkt, das gemäss GC ca. 90%ig rein ist und ein (l)/(u)-Verhältnis von ca. 1:1 aufweist.

### Beispiel 11

### Herstellung von 4-Chlor-2-methyl-2-butennitril aus 3,4-Dichlor-2-methylbutannitril

In einem mit Magnetrührer versehenen 1 l -Rundkolben werden unter Argon 24,0 g (0,15 Mol) (l,u)-3,4-Dichlor-2-methylbutannitril [(l) + (u) = 94,5 %, gemäss GC; Diastereomerenverhältnis (l) : (u) = 1: 1, gemäss GC und ¹H-NMR), 450 ml n-Pentan und 150 ml (0,3 Mol) 2N Natronlauge vorgelegt. Das Gemisch wird 2 Stunden bei Raumtemperatur unter Argon gerührt. Die Pentan-Lösung wird viermal mit je 50 ml, insgesamt 200 ml, Wasser neutral gewaschen, über wasserfreiem Natriumsulfat getrocknet, filtriert und im Rotationsverdampfer (Badtemperatur ca. 15°C, Aceton/ Trockeneis-Kühlung) eingedampft. Dies ergibt 18,5 g rohes (E/Z)-4-Chlor-2-methyl-2-butennitril als farbloses Oel, das man anschliessend über eine 10 cm lange versilberte Vigreux-Kolonne destilliert. Dies führt zu 15,6 g (88 % bezogen auf 3,4-Dichlor-2-methylbutannitril) (E/Z)-4-Chlor-2-methyl-2-butennitril als farblosem, tränenerregendem, hautschädlichem Oel, Kp. 0,1 = 20-21°C; 98%ig rein (E+Z) gemäss GC; (E)/(Z)-Verhältnis ca. 1:1,25.

### Beispiel 12

### Herstellung von (3-Cyano-2-butenyl)triphenylphosphoniumchlorid als (E/Z)-Gemisch aus (E/Z)-4-Chlor-2-methyl-2-butennitril

In einem mit Magnetrührer versehenen 25 ml -Rundkolben werden unter Argon 1,15 g (10 mMol) (E/Z)-4-Chlor-2-methyl-2-buten (E : Z = 1 : 1), 2,62 g (10 mMol) Triphenylphosphin und 7 ml Isopropanol vorgelegt. Das Reaktionsgemisch wird 60 Stunden bei Raumtemperatur unter Argon gerührt. Nach 30 Minuten Reaktionszeit geht die farblose Suspension innert ca. 2 Minuten in Lösung. Dann fällt das Produkt allmählich aus. Anschliessend werden bei Raumtemperatur 15 ml Aethylacetat zugegeben. Das Kristallisat wird unter Zugabe von weiteren 5 ml Aethylacetat filtriert, dann zweimal mit je 5 ml, insgesamt 10 ml, Diäthyläther gewaschen und 16 Stunden über wasserfreiem Phosphorpentoxid bei 22 mbar (2,2 kPa) im Exsikkator getrocknet. Dies ergibt 3,1 g (79 %) (E/Z)-(3-Cyano-2-butenyl)triphenylphosphoniumchlorid als farbloses kristallines Pulver, Smp. 276-277°C; ca. 95,8%ig (E)+(Z) rein gemäss HPLC; (E)/(Z)-Verhältnis ca. 5:1.

## Patentansprüche

1. Verfahren zur Herstellung eines (3-Alkoxycarbonyl-2-butenyl)triphenylphosphoniumchlorids oder alkylsulfats der allgemeinen Formel
X⁻(C₆H₅)₃P⁺CH₂CH=C(CH₃)COOR I
worin R eine Alkylgruppe und X⁻ das Chlor- bzw. Alkylsulfat-Ion bedeuten,
dadurch gekennzeichnet, dass man 2-Methyl-3-butennitril der Formel
CH₂=CHCH(CH₃)CN II
mittels eines Alkalimetall- oder Erdalkalimetallhypochlorits α-chloriert, und das so erhaltene 2-Chlor-2-methyl-3-butennitril der Formel
CH₂=CHC(Cl)(CH₃)CN III
entweder zuerst einer Alkanolyse mit einem Alkanol ROH, worin R eine Alkylgruppe bedeutet, zum entsprechenden 2-Chlor-2-methyl-3-butensäurealkylester der allgemeinen Formel
CH₂=CHC(Cl)(CH₃)COOR IV
unterwirft und anschliessend diesen mit Triphenylphosphin zum gewünschten (3-Alkoxycarbonyl-2-butenyl)triphenylphosphoniumchlorid der allgemeinen Formel
Cl⁻(C₆H₅)₃P⁺CH₂CH=C(CH₃)COOR I'
umsetzt,
oder zuerst mit Triphenylphosphin zum (3-Cyano-2-butenyl)triphenylphosphoniumchlorid der Formel
Cl⁻(C₆H₅)₃P⁺CH₂CH=C(CH₃)CN V
umsetzt und anschliessend dieses einer Alkanolyse mit dem Alkanol ROH in Gegenwart konzentrierter Schwefelsäure zum gewünschten (3-Alkoxycarbonyl-2-butenyl)triphenylphosphoniumalkylsulfat der allgemeinen Formel
ROSO₂O⁻(C₆H₅)₃P⁺CH₂CH=C(CH₃)COOR Iʺ
unterwirft.

2. Verfahren nach Anspruch 1, dadurch gekennzeichnet, dass man die α-Chlorierung des 2-Methyl-3-butennitrils unter Einsatz einer wässrigen Lösung von Natrium-, Kalium- oder Calciumhypochlorit und gegebenenfalls eines im wesentlichen mit Wasser nicht mischbaren, aprotischen organischen Lösungsmittels ohne Katalysator oder in Gegenwart eines als Phasentransferkatalysator dienenden quaternären Ammoniumsalzes im Temperaturbereich von etwa 0°C bis zur Raumtemperatur durchführt.

3. Verfahren nach Anspruch 2, dadurch gekennzeichnet, dass das organische Lösungsmittel ein aliphatischer Aether, ein niederes, gegebenenfalls halogeniertes Alkan, ein niederes Cycloalkan oder ein Petroläther ist.

4. Verfahren nach Anspruch 3, dadurch gekennzeichnet, dass das organische Lösungsmittel Diäthyläther, Diisopropyläther, n-Hexan, Methylenchlorid, Tetrachlorkohlenstoff oder Cyclohexan ist.

5. Verfahren nach einem der Ansprüche 2 bis 4, dadurch gekennzeichnet, dass der gegebenenfalls verwendete Phasentransferkatalysator Tetramethylammoniumchlorid oder Tetraäthylammoniumbromid ist.

6. Verfahren nach einem der Ansprüche 1 bis 5, dadurch gekennzeichnet, dass man die Alkanolyse des 2-Chlor-2-methyl-3-butennitrils oder des (3-Cyano-2-butenyl)triphenylphosphoniumchlorids mit Methanol oder Aethanol, insbesondere mit Aethanol, durchführt.

7. Verfahren nach einem der Ansprüche 1 bis 6, dadurch gekennzeichnet, dass man die Umsetzung des 2-Chlor-2-methyl-3-butensäurealkylesters bzw. des 2-Chlor-2-methyl-3-butennitrils mit Triphenylphosphin unter Verwendung eines Aromaten, insbesondere von Benzol, Toluol oder einem Xylol, eines Dialkylketons oder eines Alkansäure-alkylesters als Lösungsmittel durchführt.

8. 2-Chlor-2-methyl-3-butennitril.

9. Ein 2-Chlor-2-methyl-3-butensäure-alkylester der allgemeinen Formel
CH₂=CHC(Cl)(CH₃)COOR IV
worin R eine Alkylgruppe bedeutet.

10. (3-Cyano-2-butenyl)triphenylphosphoniumchlorid.

## Claims

1. A process for the manufacture of a (3-alkoxycarbonyl-2-butenyl)triphenylphosphonium chloride or alkyl sulphate of the general formula
X⁻(C₆H₅)₃P⁺CH₂CH=C(CH₃)COOR I
wherein R signifies an alkyl group and X⁻ signifies the chloride or alkyl sulphate ion,
which process comprises α-chlorinating 2-methyl-3-butenenitrile of the formula
CH₂=CHCH(CH₃)CN II
by means of an alkali metal or alkaline earth metal hypochlorite and subjecting the thus-obtained 2-chloro-2-methyl-3-butenenitrile of the formula
CH₂=CHC(CI)(CH₃)CN III
either firstly to an alkanolysis with an alkanol ROH, wherein R signifies an alkyl group, to give the corresponding alkyl 2-chloro-2-methyl-3-butenoate of the general formula
CH₂=CHC(CI)(CH₃)COOR IV
and subsequently reacting this with triphenylphosphine to give the desired (3-alkoxycarbonyl-2-butenyl)triphenylphosphonium chloride of the general formula
Cl⁻(C₆H₅)₃P⁺CH₂CH=C(CH₃)COOR I',
or firstly to reaction with triphenylphosphine to give (3-cyano-2-butenyl)triphenylphosphonium chloride of the formula
Cl⁻(C₆H₅)₃P⁺CH₂CH=C(CH₃)CN V
and subsequently subjecting this to an alkanolysis with the alkanol ROH in the presence of concentrated sulphuric acid to give the desired (3-alkoxycarbonyl-2-butenyl)triphenylphosphonium alkyl sulphate of the general formula
ROSO₂O⁻(C₆H₅)₃P⁺CH₂CH=C(CH₃)COOR Iʺ.

2. A process according to claim 1, wherein the α-chlorination of 2-methyl-3-butenenitrile is carried out using an aqueous solution of sodium, potassium or calcium hypochlorite and, if desired, an essentially water-immiscible, aprotic organic solvent without a catalyst or in the presence of a quaternary ammonium salt as the phase transfer catalayst in the temperature range of about 0°C to room temperature.

3. A process according to claim 2, wherein the organic solvent is an aliphatic ether, a lower, optionally halogenated alkane, a lower cycloalkane or a petroleum ether.

4. A process according to claim 3, wherein the organic solvent is diethyl ether, diisopropyl ether, n-hexane, methylene chloride, carbon tetrachloride or cyclohexane.

5. A process according to any one of claims 2 to 4, wherein the phase transfer catalyst, which is optionally used, is tetramethylammonium chloride or tetraethylammonium bromide.

6. A process according to any one of claims 1 to 5, wherein the alkanolysis of 2-chloro-2-methyl-3-butenenitrile or of (3-cyano-2-butenyl)triphenylphosphonium chloride is carried out with methanol or ethanol, especially with ethanol.

7. A process according to any one of claims 1 to 6, wherein the reaction of the alkyl 2-chloro-2-methyl-3-butenoate or of 2-chloro-2-methyl-3-butenenitrile with triphenylphosphine is carried out using an aromatic, especially benzene, toluene or an xylene, a dialkyl ketone or an alkyl alkanoate as the solvent.

8. 2-Chloro-2-methyl-3-butenenitrile.

9. An alkyl 2-chloro-2-methyl-3-butenoate of the general formula
CH₂=CHC(CI)(CH₃)COOR IV
wherein R signifies an alkyl group.

10. (3-Cyano-2-butenyl)triphenylphosphonium chloride.

## Revendications

1. Procédé de préparation d'un chlorure ou alkylsulfate de (3-alcoxycarbonyl-2-butényl)triphénylphosphonium de formule générale
X⁻(C₆H₅)₃P⁺CH₂CH=C(CH₃)COOR I
dans laquelle R représente un groupe alkyle et X⁻ l'ion chlore ou selon les cas alkylsulfate,
caractérisé en ce qu'on chlore en α le 2-méthyl-3-butènenitrile de formule
CH₂=CHCH(CH₃)CN II
au moyen d'un hypochlorite de métal alcalin ou de métal alcalino-terreux, et en ce que ou bien on soumet d'abord le 2-chloro-2-méthyl-3-butènenitrile de formule
CH₂=CHC(Cl) (CH₃)CN III
ainsi obtenu à une alcanolyse avec un alcanol ROH, dans lequel R représente un groupe alkyle, pour donner l'ester alkylique de l'acide 2-chloro-2-méthyl-3-buténoïque correspondant de formule générale
CH₂=CHC(Cl)(CH₃)COOR IV
puis on fait réagir celui-ci avec de la triphénylphosphine pour donner le chlorure de (3-alcoxycarbonyl-2-butényl)triphénylphosphonium désiré de formule générale
Cl⁻(C₆H₅)₃P⁺CH₂CH=C(CH₃)COOR I'
ou bien on le fait tout d'abord réagir avec de la triphénylphosphine pour donner le chlorure de (3-cyano-2-butényl)triphénylphosphonium de formule
Cl⁻(C₆H₅)₃P⁺CH₂CH=C(CH₃)CN V
puis on soumet celui-ci à une alcanolyse avec l'alcanol ROH en présence d'acide sulfurique concentré pour donner l'alkylsulfate de (3-alcoxycarbonyl-2-butényl)triphénylphosphonium désiré de formule générale
ROSO₂O⁻(C₆H₅)₃P⁺CH₂CH=C(CH₃)COOR Iʺ.

2. Procédé selon la revendication 1, caractérisé en ce qu'on effectue la chloration en α du 2-méthyl-3-butènenitrile en utilisant une solution aqueuse d'hypochlorite de sodium, de potassium ou de calcium et le cas échéant d'un solvant organique aprotique essentiellement non miscible à l'eau sans catalyseur ou en présence d'un sel d'ammonium quaternaire servant de catalyseur de transfert de phase dans un intervalle de température allant d'environ 0°C à la température ambiante.

3. Procédé selon la revendication 2, caractérisé en ce que le solvant organique est un éther aliphatique, un alcane inférieur, éventuellement halogéné, un cycloalcane inférieur ou un éther de pétrole.

4. Procédé selon la revendication 3, caractérisé en ce que le solvant organique est l'éther diéthylique, l'éther diisopropylique, le n-hexane, le chlorure de méthylène, le tétrachlorure de carbone ou le cyclohexane.

5. Procédé selon l'une des revendications 2 à 4, caractérisé en ce que le catalyseur de transfert de phase éventuellement utilisé est le chlorure de tétraméthylammonium ou le bromure de tétraéthylammonium.

6. Procédé selon l'une des revendications 1 à 5, caractérisé en ce qu'on effectue l'alcanolyse du 2-chloro-2-méthyl-3-butènenitrile ou du chlorure de (3-cyano-2-butényl)triphénylphosphonium avec du méthanol ou de l'éthanol, en particulier avec de l'éthanol.

7. Procédé selon l'une des revendications 1 à 6, caractérisé en ce qu'on conduit la réaction de l'ester alkylique de l'acide 2-chloro-2-méthyl-3-buténoïque ou selon les cas du 2-chloro-2-méthyl-3-butènenitrile avec de la triphénylphosphine en utilisant un composé aromatique, en particulier le benzène, le toluène ou un xylène, une dialkylcétone ou un ester alkylique d'acide alcanoïque comme solvant.

8. Le 2-chloro-2-méthyl-3-butènenitrile.

9. Ester alkylique de l'acide 2-chloro-2-méthyl-3-buténoïque de formule générale
CH₂=CHC(Cl)(CH₃)COOR IV
dans laquelle R représente un groupe alkyle.

10. Le chlorure de (3-cyano-2-butényl)triphénylphosphonium.
